Europäisches Patentamt

European Patent Office

Office européen des brevets

(19)

(11) Publication number: **0 086 610**
A1

(12) **EUROPEAN PATENT APPLICATION**

(21) Application number: 83300596.0

(22) Date of filing: 07.02.83

(51) Int. Cl.³: **C 12 P 1/04**
// (C12P1/04, C12R1/38)

(30) Priority: 15.02.82 JP 23099/82

(43) Date of publication of application: 24.08.83
Bulletin 83/34

(84) Designated Contracting States: **BE CH DE FR GB IT LI NL SE**

(71) Applicant: **Takeda Chemical Industries, Ltd., 27, Doshomachi 2-chome, Higashi-ku Osaka, 541 (JP)**

(72) Inventor: **Imada, Akira, 18-16, Kurakuen 4-bancho, Nishinomiya Hyogo 662 (JP)**
Inventor: **Kintaka, Kazuhiko, 21-9, Ushitora 2-chome, Ibaraka Osaka 567 (JP)**
Inventor: **Shinagawa, Susumu, 3-31, Higashishikiricho 6-chome, Higashiosaka Osaka 579 (JP)**

(74) Representative: **Laredo, Jack Joseph et al, Elkington and Fife High Holborn House 52/54 High Holborn, London, WC1V 6SH (GB)**

(54) Substance potentiating the activity of antibiotics and its production.

(57) A substance F3 which potentiates the activity of antibiotics, which is produced by the strain belonging to the genus Pseudomonas, and salts thereof potentiate the antibacterial activity of β-lactam antibiotics and can be used for the treatment of infections diseases caused by bacteria in mammal or domestic fowls, etc.

EP 0 086 610 A1

- 1 -

SPECIFICATION

A Substance Potentiating the Activity of Antibiotics
and its Production

Technical Field

This invention relates to a novel substance F3 which potentiates the activity of antibiotics, salts thereof, and methods of producing F3 and said salts.

Background Art

Until now, the protein SP127 has been known as a substance which potentiates the activity of antibiotics (M. Kikuchi et al.; The Journal of Antibiotics 30, 209-214, 1977), and it potentiates the antibacterial activity of macrolide antibiotics (ibid. 30, 215-220, 1974). On the other hand, clavulanic acid is konwn as a substance which prevents inactivation of β-lactam antibiotics by β-lactamases and synergistically potentiates its activity against β-lactamase producing bacteria [D. A. Leigh et al., Journal of Antimicrobial Chemotherapy, (1981)7, 229-236].

Disclosure of the Invention

This invention relates to a substance F3 which potentiates the activity of antibiotics, salts thereof, and a method of producing them.

Namely, this invention relates, in one aspect, to (1) a substance F3 which potentiates the activity of antibiotics and salts thereof, and, in another aspect, to (2) a method of producing a substance F3 which potentiates the activity of antibiotics, characterized by cultivating a strain which belongs to the genus Pseudomonas and is

- 2 -

capable of producing a substance F3 which potentiates the activity of antibiotics, in a culture medium and harvesting from the resulting culture broth the substance F3 thus produced as a metabolite.

The present inventors isolated from soil samples a large number of microorganisms for the purpose of developing new antibiotic substances and searched for antibiotics in the metabolites elaborated by these microorganisms. This exploration led them to the finding that certain strains among these isolates would produce a novel substance which potentiates antibacterial activity, that the strains belong to the genus Pseudomonas, and that a substance capable of potentiating the antibacterial activity of β-lactam antibiotics against gram-negative bacteria can be caused to accumulate in a culture medium by growing such a strain of microorganism therein. The present inventors accordingly isolated the substance, studied its physical, chemical and biological characteristics and, based on these characteristics, confirmed that this substance which potentiates the antibacterial activity is a novel active substance. They termed it "Substance F3 which Potentiates the Activity of Antibiotics".

It should first be noted that in this specification the substance F3 which potentiates the activity of antibiotics will sometimes be referred to briefly as F3.

In the present invention the F3-producing strain may be any species of microorganism which belongs to the genus Pseudomonas and is capable of elaborating F3. For example, Pseudomonas acidophila G-6302 and Pseudomonas mesoacidophila SB-72310 may be mentioned.

The strain G-6302 and the strain SB-72310 have been deposited at the Fermentation Research Institute, the Agency of Industrial Science and Technology (FRI, 1-3, Yatabecho higashi 1-chome, Tsukuba-gun, Ibaraki, Japan), Institute for Fermentation, Osaka (IFO, 17-85, Jusohonmachi 2-chome, Yodogawa-ku, Osaka, Japan) and The American Type

0086610

- 3 -

Culture Collection (ATCC, 12301 Parklawn Drive, Rockville, Maryland 20852 U.S.A.), respectively, as follows:

| | | FRI | IFO | ATCC |
|---|---|---|---|---|
| The strain G-6302 | Deposit number | FERM-P No. 4344 | IFO 13774 | ATCC 31363 |
| | Date of deposit | The 20th day of December The 52nd year of Showa (1977) | The 20th day of December The 52nd year of Showa (1977) | December 20, 1977 |
| The strain SB-72310 | Deposit number | FERM-P No. 4653 | IFO 13884 | ATCC 31433 |
| | Date of deposit | The 13rd day of September The 53rd year of Showa (1978) | The 13rd day of September The 53rd year of Showa (1978) | September 27, 1978 |

The morphological characteristics of the strain G-6302 and the strain SB-72310 are described in U.S. Patent 4,229,436 (patented on October 21, 1980) and U.S. Patent 4,225,586 (patented on September 30, 1980), respectively.

The strain belonging to the genus Pseudomonas which are employed in accordance with this invention are generally liable to vary their characteristics and behavious and tend to undergo mutation when exposed to ultraviolet light, X-rays, chemical mutagens and other artificial agents. However, even such mutants or variants can be employed only if they are able to elaborate F2 which is the contemplated substance of this invention.

In cultivating the above strains, mutants and variants, there are employed such assimilable carbon sources as glucose, sucrose, maltose, spent molasses, glycerol, oils (e.g. soybean oil, olive oil, etc.), organic acids (e.g. citric acid, succinic acid, gluconic acid, etc.). The nitrogen sources that may be employed include various organic and inorganic nitrogen compounds such as soybean meal, cottonseed meal, corn steep liquor, dried yeast, yeast extract, meat extract, peptone, urea, ammonium sulfate, ammonium nitrate, ammonium chloride, ammonium phosphate, etc. There are also employed such inorganic salts as are commonly used in the cultivation of bacteria, such as sodium chloride, calcium chloride, calcium carbonate, magnesium sulfate, monopotassium phosphate, disodium phosphate, etc. It was found that sulfur compounds which the strains can utilize, i.e. inorganic sulfur compounds such as sodium sulfate, sodium thiosulfate, etc. and organic sulfur compounds such as cystine, cysteine, methionine, etc. would lead to an increased yield of F3. Particularly preferred are cysteine and sodium thiosulfate. The concentration of such sulfur compounds is 0.01 to 1.0 w/v % and preferably 0.02 to 0.5 w/v %. The addition of a sulfur compound to the medium results in an increased yield of F3 and is therefore very advantageous in commercial-scale production.

If necessary, there are also incorporated in the medium heavy metal compounds such as ferrous sulfate, tin sulfate, etc. and vitamins such as vitamin $B_1$, biotin, etc. It is also possible or advantageous to add an antifoam or/and a surfactant, such as silicone oil, polyalkylene glycol ether, etc. In addition, organic or/and inorganic materials that will promote production of F3 may also be incorporated in suitable amounts.

The cultivation can be carried out either in solid medium or in liquid medium by a conventional antibiotics production method. While the cultivation in liquid medium may be conducted under standing still or with stirring, shaking or aeration, spinner culture under aeration is especially preferred. The cultivation is performed preferably at a temperature within the range of about 15°C-35°C, at a pH of the medium within the range of about 4-8, generally for about 8-168 hours, preferably for 24-144 hours.

- 5 -

The thus-produced substance F3, which is present for the most part in the culture broth filtrate, is advantageously isolated and purified from the supernatant following separation of the culture broth into a supernatant and bacterial cells by centrifugation or filtration. It is also possible to isolate and purify substance F3 directly from the culture broth.

The potency of substance F3 can be determined, for instance, by the cup method of paper disc method using bouillon agar containing 0.05 µg/ml of cefmenoxime with Escherichia coli IFO 12734 as the test organism.

The substance F3 which potentiates the activity of antibiotics can be harvested by any means commonly used for harvesting metabolites produced by microorganisms. Thus, for example, bacterial cells are removed by centrifugation and the filtrate is subjected to a common process known for isolation and purification of effective substances. Thus, there are utilized solubility or solubility difference in an adequate solvent, difference in manner or rate of precipitation from a solution, difference in affinity with a variety of adsorbents, ion exchange chromatography with an ion exchanger, concentration under reduced pressure, lyophilization, crystallization, recrystallization, drying and other means, either alone or in combination in an optional order, either singly or repeatedly.

A specific example of recovering the metabolite is as follows. The culture broth after completion of the cultivation is filtered, the filtrate is passed through an activated carbon column, and the thus-adsorbed substance F3 is eluted with a hydrophilic organic solvent system. The hydrophilic organic solvent system is, for example, a mixed solution composed of water and one or more of lower ketones, such as acetone, methyl ethyl ketone and methyl isobutyl ketone, and lower alcohols, such as methanol, ethanol, isopropanol, propanol and butanol. Since the desired substance is an acidic substance, the ion exchange resin is advantageously a Cl-type one, such as an anion exchange resin (Amberlite IRA-400, 402, Rohm & Haas, USA; Dowex-1, Dow Chemical, USA; Diaion SA-21A, Mitsubishi Chemical, Japan). The thus-adsorbed active substance, F3, is eluted with an aqueous solution of sodium chloride, for instance. For desalting, the eluate is again subjected to activated carbon column chromatography. The eluate containing the active substance is concentrated, then acetone or the like is added, and the precipitate is collected by filtration, washed with acetone, ether or the like and dried to give a pale brown powder. For further purification of the powder, column chromatography with QAE Sephadex (Pharmacia, Sweden) is advantageous. Thus, after washing QAE Sephadex A-25 is washed with phosphate buffer (pH 6.6), an aqueous solution of the above powder is passed through the column for adsorption, and the column is washed with the same buffer and then eluted with the buffer containing 0.5 w/v % of sodium chloride. The active fractions are pooled, adjusted to pH 3.0 and again passed through an activated carbon column. The column is rinsed with water and elution is performed with 50 v/v % aqueous methanol. The active fractions are pooled, concentrated under reduced pressure and lyophilized to give a powder of F3. This product is capable of forming metal and ammonium salts. The metal salts may for example be sodium, potassium and lithium salts.

The physico-chemical properties of F3 monosodium salt as obtained in Example 1 are as follows.

1. Appearance of the substance:  White powder

2. Elemental analysis:  As dried in vacuo over phosphorus pentoxide at 40°C for 6 hrs. (%)

    C   32.08
    H   5.33
    N   5.17
    S   6.28
    Na  4.60

3. Molecular weight:  520 ± 60 as calculated from the sodium content of monosodium salt.

Molecular formula (based on the above analysis)

$C_{14}H_{23}N_2O_{12}SNa \cdot (3H_2O)$

4. Ultraviolet absorption spectrum:

End absorptions only (no characteristic at wavelength over 210 nm)

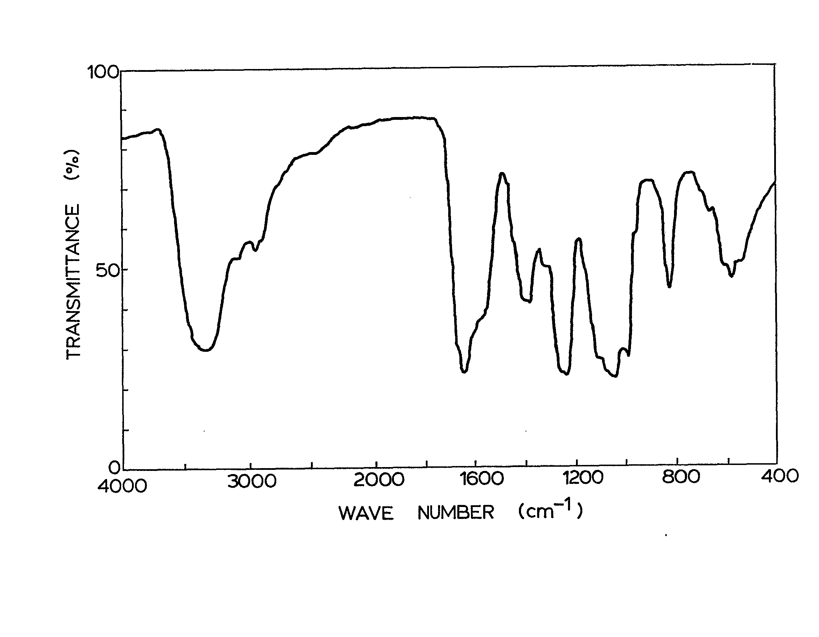

5. Infrared absorption spectrum (Fig. 1):

The dominant peaks on the absorption spectrum (KBr disk) are as follows.

3350, 3100(sh), 2950, 1660(sh), 1640, 1560(sh), 1410, 1375, 1320(sh), 1260, 1240, 1110, 1070(sh), 1030, 985, 950(sh), 820, 610, 585 $(cm^{-1})$

6. Specific rotation $[\alpha]_D^{25}$ -2.8° (C=0.5, 0.1 M-$Na_2HPO_4$)

7. Solubility:

Insoluble in petroleum ether, hexane, diethyl ether, benzene, ethyl acetate and chloroform; only sparingly soluble in ethanol, pyridine and acetone; soluble in methanol and dimethyl sulfoxide; and readily soluble in water.

- 8 -

8. Color reactions:

Positive Greig-Leaback, ninhydrin and potassium permanganate reactions.  Negative ferric chloride-potassium ferricyanide reactions.

9. Stability:

Stable in aqueous solution at pH 3 to 9 against heating 60°C for 10 minutes.

The bilogical properties of the substance F3 which potentiates the activity of antibiotics are as follows. It will be apparent that, in the co-presence of F3, cefmenoxime and mecillinam kill Escherichia coli at one-half of the respective killing doses.

Table 1  The action of F3 of Example 1 to potentiate the antibacterial action of cefmenoxime and mecillinam. (Test organism: Escherichia coli IFO 12734)

Table 1

| Concentration of F3 in test solution ** ($\mu$g/ml) | Diameter of growth inhibition zone (mm)* | | |
|---|---|---|---|
| | No addition | 0.05 $\mu$g/ml of cefmenoxime | 0.03 $\mu$g/ml of mecillinam |
| 0 | <8 | <8 | <8 |
| 1 | <8 | 10 | <8 |
| 5 | <8 | 12.5 | <8 |
| 25 | <8 | 15 | 9 |
| 125 | <8 | 26 | 17.5 |
| 1000 | <8 | 38 | 28.5 |

* The test was performed using a bouillon agar medium containing the antibiotic as specified and a paper disc, 8 mm in diameter.

** A paper disc, 8 mm in diameter, was impregnated with 25 $\mu$l of the test solution and placed on the agar medium

- 9 -

Further, it is apparent from Table 2 that F3 potentiates the inhibitory action of cefmenoxime and mecillinam against _Proteus mirabilis_ ATCC 21100 as well.

Table 2  The action of F3 of Example 1 to potentiate the antibacterial action of cefmenoxime and mecillinam*

(test organism:  _Proteus mirabilis_ ATCC 21100)

Table 2

| Concentration of F3 in test solution (µg/ml) | Diameter of growth inhibition zone (mm)* | | |
|---|---|---|---|
| | No addition | 0.01 µg/ml of cefmenoxime | 0.05 µg/ml of mecillinam |
| 0 | <8 | <8 | <8 |
| 1 | <8 | 9 | <8 |
| 5 | <8 | 12 | 9 |
| 25 | <8 | 21 | 13.5 |
| 125 | <8 | 28.5 | 19 |
| 1000 | <8 | 32.5 | 30 |

* Except for the test organism and the concentration of antibiotics to be potentiated, all other assay conditions are the same as those mentioned for Table 1.

Furthermore, F3 strongly inhibits the growth of _Escherichia coli_ in synergy with cefmenoxime.  In Table 3, there are shown degrees of growth of _Escherichia coli_ under the influence of F3 in the copresence of 1 µg/ml of cefmenoxime.

Table 3 Inhibition of growth of Escherichia coli IFO 12734:
in the co-presence of F3 (Example 1) and cefmenoxime

Table 3

| Final concentra-tion of F3 (μg/ml) | Absorbance at 600 nm | |
|---|---|---|
| | No addition | 1 μg/ml of cefmenoxime |
| 0 | 3.7 | 2.9 |
| 1 | 3.7 | 2.2 |
| 10 | 3.7 | 0.6 |
| 100 | 3.7 | 0.6 |

To 8 ml of YAB medium containing 12 % (w/v) of
sucrose (Difco, Antibiotic Medium No. 3, 1.75 %; Difico
yeast extract 0.5 %) were added water, 1 ml of 10  g/ml
cefmenoxime and 1 ml of a culture of the organism grown
to an early stage of logarithmic phase in YAB medium.
The mixture was shake-cultured at 37°C for 2 hours, at
the end of which time an absorbance measurement was made
at 600 nm using a Shimadzu-Voshrom Spectronic 20 Colorimeter.

As is evident from Table 3, F3 inhibits the growth of
Escherichia coli in the presence of cephalexin or cefmenoxime,
as evidenced by the decrease in absorbance. Observation of
the culture broths under a phase contrast microscope revealed that, when F3 was not added, cells were in the much
elongated form under the influence of cephalexin or
cefmenoxime but that, when F3 was added, the elongation was
inhibited in dependence on the addition level and at the
same time partial swelling occurred and bacteriolysis
proceeded.

The substance F3 which potentiates the activity of
antibiotics obtainable by the method of the present invention

- 11 -

showed a marked antibacterial activity in synergy with cefmenoxime or cephalexin in Escherichia coli-infected mice. Therefore, F3 can be used in combination with such antibiotics in the treatment of infectious diseases caused by the above bacteria in mammals (e.g. mouse, rat, dog, human) and in domestic fowls (e.g. chicken, duck).

For the treatment of Escherichia coli infection, for instance, a solution of 0.5-2.0 g of, for example, cefmenoxime and 0.5-10 g of F3 in 10-100 ml of physiological saline is administered to human adults two to three times a day in admixture with an injectable solution for intravenous drip infusion.

F3 is also a very promising intermediate for the synthesis of novel drugs.

When F3 was intravenously administered to mice, the $LD_{50}$ was more than 1 g/kg.

For comparison of the substance F3 which potentiates the activity of antibiotics, which has the above properties, there may be mentioned first of all such water-soluble, S-containing, acidic antibiotics as penicillins and cephalosporins. In contrast with these, substance F3 shows no absorption at wavelengths over 1720 $cm^{-1}$ in the infrared absorption spectrum and, when tested in a usual manner, does not show any antibacterial activity. Therefore, F3 is different from said antibiotics.

Moreover, while many antibiotics are known which are produced by Pseudomonas bacteria, F3 differs from all of them in physical, chemical and biological properties. It is, therefore, considered that F3 is a novel compound.

The following examples are further illustrative but not limitative of this invention. The percents given in the examples are by weight/volume unless otherwise stated.

- 12 -

Example 1

Cells of Pseudomonas mesoacidophila SB-72310
(FERM-P No. 4653; IFO 13884; ATCC-31433) were inoculated
into two 2-liter Sakaguchi flasks each containing 500 ml
of a medium composed of 1% glucose, 0.5% Polypepton
(Daigo Eiyo Chemical Co., Ltd.), 0.5% meat extract and
0.5% sodium chloride (pH 7.0) and the inoculated flasks
were incubated on the reciprocating shaker at 28°C for
48 hours to prepare a seed inoculum.

Then a 200-liter stainless steel fermentor was
charged with 120 ℓ of a medium composed of 3% glycerol,
0.1% glucose, 0.5% polypeptone, 0.5% meat extract, 0.5%
NaCl and 0.1% cysteine, the pH of which was adjusted to
pH 7 with 30% sodium hydroxide. The fermentor was steam-
sterilized at 120°C for 20 minutes, after which the
medium was inoculated with the seed inoculum. Cultivation
was conducted at 28°C with 120 ℓ/min. aeration and at
180 r.p.m. for 78 hours. The culture broth was centrifuged
with a Sharples sentrifuge to separate the cells to recover
110 ℓ of a supernatant. It was adjusted to pH 4.2 and
passed through a column of 15 ℓ activated carbon
(Chromatography grade Shirasagi, Takeda Chemical Industries,
Ltd.) to adsorb the activity. The column was rinsed with
45 ℓ of water and elution was carried out with 45 ℓ of
50 v/v% aqueous acetone. The eluate was collected in 10 ℓ
fractions and the active fractions were detected using
plates of broth agar containing 0.05 g/ml of cefmenoxime
and, as the test organism, Escherichia coli IFO 12734.
Fraction Nos. 2 and 3 were pooled, diluted with 20 ℓ of
water and passed through a column of 10 ℓ Dowex-1(Cl)
(Dow and Chemical, U.S.A.). After the column was rinsed
with 24 ℓ of water, elution was carried out with 20 v/v%
aqueous methanol. The active fraction was concentrated
under reduced pressure to 50 ml, followed by addition of
200 ml of acetone. The resultant precipitate was
recovered by filtration, washed with 50 ml of acetone and 100 ml of ether, and dried in vacuo to give 21 g of a crude product.

Ten (10) grams of this crude product was dissolved in 500 ml of 1/100 M-phosphate buffer (pH 6.6) and passed through a column of 200 ml QAE-Sephadex A-25 buffered with the same buffer as above. The column was washed with 1000 ml of the same buffer as above and eluation was carried out with 2000 ml of the same buffer containing 0.5% of sodium chloride. The fractions showing F3 activity were pooled, adjusted to pH 3.0 with 1N-HCl, and passed through a column of 40 ml activated carbon. The column was rinsed with 200 ml of water and elution was carried out with 50 v/v% aqueous methanol. The active fractions were pooled, concentrated under reduced pressure and lyophilized. The white powder thus obtained was dried over phosphorus pentoxide under reduced pressure at 40°C for 6 hours to give 0.85 g of a powder. The infrared absorption spectrum (KBr) of this powder is shown in Fig. 1.

Elemental analysis: C, 32.08; H, 5.33; N, 5.17; S, 6.28;
                    Na, 4.60%

Example 2

Cells of Pseudomonas acidophila G-6302 (FERM-P No. 4344; IFO 13774; ATCC-31363) grown on a nutrient broth agar slant were used to inoculate two 2-liter Sakaguchi flasks each containing 500 ml of a medium composed of 1% glucose, 0.5% Polypepton, 0.5% meat extract and 0.5% sodium chloride (pH 7.0) and the inoculated medium was incubated on a reciprocating shaker at 28°C for 48 hours to give a seed inoculum.

Separately, a 200-liter stainless steel fermentor was charged with 120 ℓ of a medium composed of 3% glycerol, 0.1% glucose, 0.5% Polypepton, 0.5% meat extract, 0.5% NaCl and 0.1% cysteine and after adjustment to pH 7.0 with 30% sodium hydroxide, was steam-sterilized at 120°C for 20 minutes. The fermentor was then inoculated

- 14 -

with the above seed inoculum and incubation was carried
out at 28°C and 180 r.p.m. with 120 ℓ/min. aeration for
78 hours. The resulting broth was centrifuged with
a Sharples centrifuge to separate the cells and give
110 ℓ of a supernatant. This supernatant was adjusted
to pH 4.2 and passed through 15 ℓ of activated carbon
(Chromatography grade Shirasagi, Takeda Chemical Industries,
Ltd.). After an aqueous rinse with 45 ℓ of water, elution
was carried out with 45 ℓ of 50 v/v% aqueous acetone.
The eluate was collected in 10-liter fractions which were
assayed for activity using plates of broth-agar containing
0.05 μg/ml of cefmenoxime and, as the test organism,
Escherichia coli IFO 12734. Fraction Nos. 2 and 3 were
pooled, diluted with 20 ℓ of water and passed through a
column of 10 ℓ Dowex-1(Cl) (Dow and Chemical, U.S.A.).
The column was rinsed with 25 ℓ of water and elution was
carried out with 50 ℓ of 5% aqueous sodium chloride. The
active fractions were pooled, adjusted to pH 4.0 and
passed again through a column (8ℓ) packed with activated
carbon. After the column was rinsed with 24 ℓ of water,
elution was carried out with 20 v/v% aqueous methanol. The
active fractions were pooled and concentrated under
reduced pressure to 50 ml. To the residue was added
200 ml of acetone and the resultant precipirate was
recovered by filtration, washed with 50 ml of acetone
and 100 ml of ether, and dried in vacuo to give 25 g
of a crude product.

A 10 g portion of the crude product was dissolved
in 500 ml of M/100 phosphate buffer (pH 6.6) and adsorbed
on a column of 200 ml QAE-Sephadex A-25 buffered with the
same buffer as above. The column was washed with 1000 ml
of the same buffer as above and elution was carried out
with 2000 ml of the same buffer containing 0.5% of sodium
chloride. Fractions containing F3 activity were pooled,
adjusted to pH 3.0 with 1N-HCl, and passed through a column of activated carbon.  The column was rinsed with
200 ml of water and elution was carried out with 50 v/v%
aqueous methanol.  The active fractions were pooled,
concentrated under reduced pressure, and lyophilized.
The resulting white powder was dried in vacuo over
phosphorus pentoxide at 50°C for 6 hours to give 1.3 g
of a powder.
Elemental analysis:  C, 32.28; H, 5.53; N, 5.18; S, 6.25;
                     Na, 4.52%

Brief Description of the Drawing

    Fig. 1 is an infrared absorption spectrum (KBr)
of the antibiotic potentiating agent F3 of Example 1.

-16-

CLAIMS

1.    A substance F3 which potentiated the activity of antibiotics, inclusive of salts thereof, which as a monosodium salt thereof has the following physical and chemical properties:

(1)    Appearance: white powder

(2)    Elemental analysis (%) (as dried over phosphorus pentoxide at 40°C for 6 hours):

      C     32.30 ± 1.0

      H      5.60 ± 0.5

      N      5.38 ± 0.5

      S      6.16 ± 1.0

      Na    4.42 ± 0.5

(3)    Ultraviolet absorption spectrum: No characteristic absorptions at wavelengths $\geq$ 210 nm

(4)    Infrared absorption spectrum: The dominant absorptions (wave-numbers) in the infrared region as measured by the potassium bromide disk method are as follows. 1640, 1260, 1240, 1030, 985, 820 ($cm^{-1}$)

(5)    Specific rotation: $[\alpha]_D^{25}$ - 2.8°±2° (c = 0.5, 0.1 M-$Na_2HPO_4$)

(6)    Solubility: Insoluble in petroleum ether, hexane, diethyl ether, benzene, ethyl acetate and chloroform; hardly soluble in ethanol, pyridine and acetone; soluble in methanol and dimethyl sulfoxide; and readily soluble in water.

(7)    Molecular weight: 520±60

(8)    Color reactions: Positive Greig-Leaback, ninhydrin and potassium permanganate reactions; and negative ferric chloride-potassium ferricyanide and Sakaguchi reactions.

2.    A method of producing a substance F3 which potentiates the activity of antibiotics, characterized by cultivating a microorganism which belongs to the genus <u>Pseudomonas</u> and is capable of producing a substance F3 which potentiates the activity of antibiotics, in a culture medium and harvesting from the resulting culture broth the substance F3 thus produced as a metabolite.

3.    The method according to Claim 2, wherein the microorganism is <u>Pseudomonas</u> <u>acidophila</u>.

4.    The method according to Claim 2, wherein the microorganism is <u>Pseudomonas</u> <u>acidophila</u> G-6302 (IFO 13774).

5.    The method according to Claim 2, wherein the microorganism is <u>Pseudomonas</u> <u>mesoacidophila</u>.

6.    The method according to Claim 2, wherein the microorganism is <u>Pseudomonas</u> <u>mesoacidophila</u> SB-72310 (IFO 13884).

European Patent
Office

**EUROPEAN SEARCH REPORT**

Application number

EP 83 30 0596

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-2 011 380 (TAKEDA) <br> *The whole document* <br><br> --- | 1,2 | C 12 P 1/04 // <br> (C 12 P 1/04 <br> C 12 R 1/38 ) |
| A | DE-A-2 939 333 (TAKEDA) <br><br> *The whole document* <br><br> ----- | 1,2,5, 6 | |

TECHNICAL FIELDS
SEARCHED (Int. Cl. 3)

C 12 P 1/00
C 12 R 1/00

The present search report has been drawn up for all claims

| Place of search <br> THE HAGUE | Date of completion of the search <br> 01-06-1983 | Examiner <br> RAJIC M. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82